Europäisches Patentamt

European Patent Office

Office européen des brevets

(19)

(11) Veröffentlichungsnummer: **0 256 522**
**A2**

(12) # EUROPÄISCHE PATENTANMELDUNG

(21) Anmeldenummer: **87111735.4**

(22) Anmeldetag: **13.08.87**

(51) Int. Cl.⁴: **A61K 37/00**

(30) Priorität: **16.08.86 DE 3627762**

(43) Veröffentlichungstag der Anmeldung:
**24.02.88 Patentblatt 88/08**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Löbermann, Hartmut, Dr.**
**Giessener Strasse 50**
**D-3556 Weimar(DE)**
Erfinder: **Rainer, Peter**
**Otto-Ubbelohde-Weg 31**
**D-3551 Lahntal(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) **Verfahren zur Herstellung einer prothrombin-armen Präparation Vitamin K-abhängiger Gerinnungsfaktoren, sowie ein danach erhältliches Mittel.**

(57) Es wird ein Verfahren zur Herstellung einer Präparation prothrombin-armer Gerinnungsfaktoren beschrieben, das dadurch gekennzeichnet ist, daß zu einer Lösung, die Prothrombin und mindestens einen weiteren Gerinnungsfaktor sowie einen Puffer enthält, Thrombin zugesetzt, das Thrombin einwirken gelassen, das Thrombin abgetrennt und der Gerinnungsfaktor gewonnen wird.

Eine auf diese Weise hergestellte Präparation eines Gerinnungsfaktors kann zur Therapie von Gerinnungsstörungen verwendet werden.

EP 0 256 522 A2

## Verfahren zur Herstellung einer prothrombin-armen Präparation Vitamin K-abhängiger Gerinnungsfaktoren, sowie ein danach erhältliches Mittel

Die Erfindung betrifft ein Verfahren zur Herstellung einer prothrombin-armen Präparation von Gerinnungsfaktoren, sowie ein danach erhältliches Mittel.

Vitamin K-abhängige Gerinnungsfaktoren werden zur Therapie haemostatischer Störungen verwendet. Die Anwendung entsprechender Gerinnungsfaktorenkonzentrate ist zum Teil mit unerwünschten Nebenreaktionen verbunden, die zum Teil auf eine teilweise Prothrombinaktivierung zurückzuführen sind.

Aufgabe der Erfindung ist daher die Entwicklung eines Verfahrens zur Herstellung prothrombin-armer vitamin K-abhängiger Gerinnungsfaktoren, sowie ein danach erhältliches Mittel. Die Abtrennung des Prothrombins sollte selektiv und schonend erfolgen, ohne die anderen Gerinnungsfaktoren in ihrer Aktivität zu beeinträchtigen.

Unter anderen Gerinnungsfaktoren sind besonders die vitamin K-abhängigen Plasmaproteine, insbesondere Faktor II, Faktor VII, Faktor IX, Faktor X, Protein C, Protein S sowie Protein Z zu verstehen. Es ist bekannt, daß Prothrombin durch limitierte Proteolyse mittels Thrombin bei pH 7,5 bis 8 in Fragmente abgebaut werden kann (Arch. Biochem. Biophys. (1985) 240 (2), 607-612).

Es wurde überraschenderweise gefunden, daß ein thrombinkatalysierter Abbau des Prothrombins zur Herstellung prothrombin-armer Gerinnungsfaktoren verwendet werden kann.

Es wurde weiter überraschenderweise gefunden, daß ein selektiver Prothrombinabbau in einem geeigneten Puffersystem unter Einhaltung eines geeigneten pH-Wertes und gegebenenfalls durch Zusatz chemischer Substanzen ohne Beeinträchtigung der anderen Gerinnungsfaktoren möglich ist.

Es wurde weiterhin überraschenderweise gefunden, daß die durch Thrombineinwirkung entstandenen Prothrombinfragmente durch Adsorption vorzugsweise an schwerlösliche Calcium-, Barium-oder Aluminium-Salze, Anionenaustauscher oder trägergebundenes Heparin oder Dextransulfat von den anderen Gerinnungsfaktoren weitestgehend abgetrennt werden können. Die Gerinnungsfaktoren können so in höherer Ausbeute gewonnen werden.

Gegenstand der Erfindung ist somit ein Verfahren zur Herstellung einer Präparation prothrombin-armer Gerinnungsfaktoren, sowie ein danach erhältliches Mittel.

Dieses Verfahren ist dadurch gekennzeichnet, daß zu einer wässrigen Lösung, die Prothrombin und mindestens einen weiteren Gerinnungsfaktor sowie einen Puffer enthält, Thrombin zugesetzt, das Thrombin einwirken gelassen, das Thrombin abgetrennt, die durch Thrombineinwirkung entstandenen Prothrombinfragmente durch Adsorption vorzugsweise an schwerlösliche Calcium-, Barium-oder Aluminium-Salze, Anionenaustauscher oder trägergebundenes Heparin oder Dextransulfat von den anderen Gerinnungsfaktoren weitestgehend abgetrennt und die Gerinnungsfaktoren gewonnen werden.

Das Thrombin wird vorzugsweise in einem 0,001-1 mol/l Na-oder K-Phosphat-, Na-oder K-Citrat-, Glycin-, Imidazol-oder Na-Acetat-Puffer bei einem pH-Wert von 5-7, einer Temperatur von 0-45°C sowie gegebenenfalls unter Zusatz weiterer Substanzen, beispielsweise 0,001-3 mol/l NaCl, LiCl oder KCl, 0-0,1 mol/l EDTA oder 1-40 g/100 ml Ammoniumsulfat, für eine Dauer von 5 bis 500 min einwirken gelassen.

Besonders bevorzugt wird das Thrombin in einem 0,01-0,3 mol/l Na-Phosphat-oder Imidazolpuffer oder einem 0,001-0,05 mol/l EDTA-Puffer bei einem pH-Wert von 5,5-6,5, einer Temperatur von 20-40°C für eine Dauer von 30-180 min einwirken gelassen.

In einer besonders vorteilhaften Ausführungsform wird das Thrombin zum Beispiel in einer Lösung mit 0,02 mol/l Imidazol und 0,001 mol/l EDTA, pH 6 oder mit 0,025 mol/l K-Phosphat pH 6, bei etwa 37°C für die Dauer von etwa 120 min einwirken gelassen.

Als Thrombin kann besonders, gegebenenfalls trägergebundenes, Human-oder Rinder-Thrombin in einer Konzentration von 0,1-5000 U/ml Lösung verwendet werden. 2500 Einheiten Thrombin entsprechen 1 mg Proteinsubstanz.

Bevorzugt wird aber gegebenenfalls trägergebundenes Human-Thrombin in einer Konzentration von 1-300 U/ml.

In einer ganz bevorzugten Ausführungsform wird trägergebundenes Human-Thrombin in einer Konzentration von 5-30 U/ml Lösung verwendet.

Eine Einheit (U) Thrombin entspricht der Menge an Enzym, die bei einem pH-Wert von 7,5 und 37°C durch Spaltung des chromogenen Substrates Tos-Gly-Pro-Arg-pNA 1 OD/ml.min bei 405 nm erzeugt.

Die Bindung des Thrombins an das Trägermaterial wird nach in der Literatur beschriebenen Verfahren z.B. nach Cuatrecasas, P., Wilchek, M., Anfinsen, C.B. (1968) Proc. Natl. Acad. Sci. USA, 61, 636-643 durchgeführt, wobei Thrombin beispielsweise in einer Konzentration von 10-5000, vorzugsweise 50-1000 μg pro ml CNBr-oder epoxyaktivierter [R]Sepharose oder [R]Agarose eingesetzt wird.

In einer bevorzugten Ausführungsform werden 100-500 μg Thrombin mit einem ml CNBr-aktivierter ᴿSepharose umgesetzt.

Zur Gewinnung der Gerinnungsfaktoren können sie nach Entfernung des Thrombins aus der Lösung an schwerlösliche Calcium-, Barium-oder Aluminiumsalze oder einen Anionenaustauscher oder trägergebundenes Heparin oder Dextransulfat adsorbiert werden. Die Lösung wird vom Adsorptionsmaterial getrennt, das Adsorbens gegebenenfalls gewaschen und die Gerinnungsfaktoren eluiert.

Dazu wird die Lösung, gegebenenfalls nach Zusatz von 0,001-3 mol/l eines löslichen Alkalisalzes, besonders NaCl, KCl oder LiCl, oder 1-40 g/100 ml Ammoniumsulfat, bei einem pH-Wert von 5-9 mit einem anorganischen Adsorbens, beispielsweise Tri-Calciumphosphat, Hydroxylapatit, BaSO₄ oder Al(OH)₃ oder einem Anionenaustauscher, beispielsweise DEAE-ᴿSephadex, DEAE-ᴿSephacel oder DEAE-ᴿSepharose, oder trägergebundenem Heparin oder Dextransulfat, beispielsweise Heparin-ᴿSepharose oder Dextransulfat-ᴿSepharose in Kontakt gebracht.

In einer bevorzugten Ausführungsform werden mindestens 0,01 g Tri-Calciumphosphat, BaSO₄ oder Al-(OH)₃ pro ml Lösung oder beispielsweise auch mindestens 0,01 g Hydroxylapatit pro ml Lösung bei einem pH-Wert von 6-7 gegebenenfalls nach Zusatz von 5 bis 35 g/100 ml Ammoniumsulfat oder 1-3 mol/l NaCl verwendet.

In einer ganz besonders bevorzugten Ausführungsform wird die Lösung entweder mit 10-20 g/100 ml Ammoniumsulfat versetzt und bei einem pH-Wert von 6-6,5 mit Tri-Calciumphosphat oder aber ohne Zusätze bei einem pH-Wert von 6-6,5 mit Hydroxylapatit in Kontakt gebracht. Sollte sich durch den Zusatz von Ammoniumsulfat ein Niederschlag bilden, so ist dieser vor der Adsorption, beispielsweise durch Zentrifugation oder Filtration, von der Lösung abzutrennen.

Anschließend wird das Adsorbens von der Lösung abgetrennt und gegebenenfalls mit einer Pufferlösung, beispielsweise 0,001-0,5 mol/l Na-oder K-Phosphat, 0,005-0,1 mol/l Imidazol, 0,001-0,02 mol/l Na-oder K-Citrat oder 0,001-0,03 mol/l EDTA bei einem pH-Wert von 5,5-7, bevorzugt mit 0,005-0,05 mol/l Imidazol oder 0,001-0,03 mol/l Na-Citrat, pH 6,0-6,8, besonders bevorzugt mit etwa 0,02 mol/l Imidazol oder 0,01-0,015 mol/l Na-Citrat, pH 6, gewaschen. Bei Verwendung von Hydroxylapatit als Adsorbens wird dieses beispielsweise mit 0,001-0,5 mol/l Na-oder K-Phosphat, 0,001-0,02 mol/l Na-oder K-Citrat bei einem pH-Wert von 5,5-7, bevorzugt mit 0,01-0,05 mol/l K-Phosphat, pH 6-6,5, besonders bevorzugt mit etwa 0,025 mol/l K-Phosphat pH 6 und danach mit etwa 0,16 mol/l K-Phosphat pH 6,2 gewaschen. Anschließend werden die Gerinnungsfaktoren nach einem bekannten Verfahren beispielsweise nach Thromb. Diath. Haemorrh. Supp. (1969) 35, 6l, eluiert.

Ein nach den beschriebenen Verfahren gewonnenes Gerinnungsfaktorenpräparat zeichnet sich dadurch aus, daß es prothrombin-arm ist und die ursprünglichen biologischen Aktivitäten der Gerinnungsfaktoren kaum beeinträchtigt sind.

Eine solche Präparation kann nach an sich bekannten Verfahren beispielsweise durch Erhitzung, Dialyse, Sterilfiltration und Lyophilisation weiterverarbeitet werden und gegebenenfalls in Form eines Mittels, das blutisotonisch, bakterizid und virusfrei gemacht worden ist und gegebenenfalls physiologisch verträgliche Zusätze und Stabilisatoren enthält,als Arzneimittel verabreicht werden.

Die folgenden Beispiele erläutern die Erfindung:

## Beispiel 1

### Herstellung eines prothrombin-armen Faktor IX-, X-, Protein C-und S-Konzentrates:

50 ml einer Lösung, die 30 Einheiten pro ml der Gerinnungsfaktoren II, IX, X sowie Protein C und S enthält, wurden gegen einen Puffer von pH 6, der 20 mmol/l Imidazol/HCl und 1 mmol/l EDTA enthielt, dialysiert. Eine Einheit Gerinnungsfaktor entspricht der Aktivität des jeweiligen Gerinnungsfaktors in 1 ml Citrat-Normalplasma. Nach der Dialyse wurden 0,4 ml Thrombin-ᴿSepharose zugesetzt (1 ml Thrombin-ᴿSepharose enthielt 500 μg gebundenes Human-Thrombin).

Die Lösung wurde anschließend 2 Stunden bei 37°C gerührt, durch Filtration von der Thrombin-ᴿSepharose befreit und auf eine Ammoniumsulfat-Konzentration von 17 g/100 ml gebracht. Falls sich ein Niederschlag gebildet hatte, wurde dieser durch Zentrifugation abgetrennt und verworfen. Der Überstand wurde mit 0,15 g Tri-Calciumphosphat pro ml Lösung versetzt und 30 min bei Raumremperatur gerührt. Nach Zentrifugation wurde der Feststoff 4-mal mit 100 ml einer Lösung, die 20 mmol/l Imidazol/HCl und 10 mmol/l Na-Citrat enthielt, pH 6, gewaschen und anschließend mit 100 ml einer Lösung, die 20 mmol/l Imidizol/HCl und 250 mmol/l K-Citrat enthielt, pH 6.5, eluiert.

GERINNUNGSAKTIVITÄTEN

|   | Faktor II | | Faktor IX | | Faktor X | | Protein C | |
|---|---|---|---|---|---|---|---|---|
| A | 30 | E/ml | 28 | E/ml | 27 | E/ml | 22 | E/ml |
| B | 0,9 | E/ml | 25,6 | E/ml | 24,8 | E/ml | 20,5 | E/ml |
| C | 0,15 | E/ml | 12,2 | E/ml | 11,8 | E/ml | 9,8 | E/ml |

A = Ausgangslösung, 50 ml, enthaltend Faktor II, Faktor IX, Faktor X, Protein C und S

B = Lösung nach Behandlung mit Thrombin-Sepharose (2 Std, 37°C), 50 ml

C = $Ca_3(PO_4)_2$-Eluat, 100 ml.

Die angegebenen Einheiten für die Aktivität der Gerinnungsfaktoren sind über eine entm rechende Gerinnungszeitbestimmung des Einzelfaktors ermittelt worden. Eine Einheit (E) entspricht der Aktivität des jeweiligen Gerinnungsfaktors in 1 ml Citrat-Normalplasma.

Beispiel 2

Verwendung einer Gerinnungsfaktorenlcsung wie in Beispiel 1, die 0,025 mol/l K-Phosphat pH 6 enthält. Zu 50 ml dieser Lösung wurden nach Behandlung mit Thrombin-[R]Sepharose (siehe Beispiel 1) 12,5 g Hydroxylapatit zugesetzt und 30 min gerührt. Das Adsorbens wurde nach Abtrennen der überstehenden Lösung mit 100 ml 25 mmol/l K-Phosphat pH 6 und anschließend mit 100 ml 160 mmol/l K-Phosphat pH 6,2 gewaschen. Die Gerinnungfaktoren wurden mit 100 ml 0,3 mol/l K-Phosphat pH 6,5 eluiert. Die Aktivitäten der einzelnen Gerinnungsfaktoren in dem Ausgangsmaterial und dem 0,3 mol/l K-Phosphat-Eluat entsprechen in etwa den Werten wie sie in der Tabelle für Beispiel 1 aufgeführt sind.

**Ansprüche**

1. Verfahren zur Herstellung einer Präparation prothrombin-armer Vitamin K-abhängiger Gerinnungsfaktoren, dadurch gekennzeichnet, daß zu einer Lösung, die Prothrombin und mindestens einen weiteren Gerinnungsfaktor sowie einen Puffer enthält, Thrombin zugesetzt, das Thrombin einwirken gelassen, das Thrombin und die durch Thrombineinwirkung entstandenen Prothrombinfragmente abgetrennt und die Gerinnungsfaktoren gewonnen werden.

2. Verfahren nach Anspruch 1, dadurch gekennzeichnet, daß man Thrombin in einem 0,001-1 mol/l Na- oder K-Phosphat-, Na-oder K-Citrat-, Glycin-, Imidazol-oder Na-Acetat-Puffer bei einem pH-Wert von 5-7, einer Temperatur von 0-45°C sowie gegebenenfalls unter Zusatz von 0,001-3 mol/l NaCl, LiCl oder KCl, 0-0,1 mol/l EDTA oder 1-40 g/100 ml Ammoniumsulfat, für eine Dauer von 5 bis 500 min einwirken läßt.

3. Verfahren nach Anspruch 1, dadurch gekennzeichnet, daß trägergebundenes Human-oder Rinder-Thrombin in einer Konzentration von 0,1-5000 U/ml Lösung verwendet wird.

4. Verfahren nach Anspruch 1, dadurch gekennzeichnet, daß die Lösung nachAbtrennung des Thrombins, gegebenenfalls nach Zusatz von 0,001-3 mol/l eines löslichen Alkalisalzes, bei einem pH-Wert von 5-9, mit einem anorganischen Adsorbens, einem Anionenaustauscher, oder trägergebundenem Heparin oder Dextransulfat in Kontakt gebracht wird.

5. Arzneimittel zur Therapie von Gerinnungsstcrungen, enthaltend einen nach Anspruch 1 erhältlichen Gerinnungsfaktor.